Europäisches Patentamt

European Patent Office

Office européen des brevets

(11) Publication number: **0 514 554 A1**

# EUROPEAN PATENT APPLICATION
## published in accordance with Art. 158(3) EPC

(21) Application number: 92900887.8

(22) Date of filing: 06.12.91

(86) International application number:
**PCT/JP91/01697**

(87) International publication number:
**WO 92/10508 (25.06.92 92/14)**

(51) Int. Cl.⁵: **C07K 3/20, G01N 30/48**

(30) Priority: **06.12.90 JP 400721/90**
**06.12.90 JP 400722/90**

(43) Date of publication of application:
**25.11.92 Bulletin 92/48**

(84) Designated Contracting States:
**CH DE FR GB LI NL SE**

(71) Applicant: **SUMITOMO ELECTRIC INDUSTRIES, LTD.**
**5-33, Kitahama 4-chome, Chuo-ku**
**Osaka-shi, Osaka 541(JP)**

(72) Inventor: **NAKABAYASHI, Makoto c/o Osaka Works Sumitomo**
**El. Ind. Ltd 1-3, Simaya 1-chome,**
**Konohana-ku**
**Osaka-shi, Osaka 554(JP)**
Inventor: **NIWA, Shinichiro c/o Osaka Works Sumitomo El. Ind.**
**Ltd. 1-3, Shimaya 1-chome Konahana-ku**
**Osaka-shi, Osaka 554(JP)**
Inventor: **KISHIMOTO, Toshihiko Osaka Works Sumitomo El. Ind.**
**Ltd. 1-3, Shimaya 1-chome Konohana-ku**
**Osaka-shi, Osaka 554(JP)**
Inventor: **UNO, Atsushi c/o Osaka Works Sumitomo El.Ind. Ltd.**
**1-3, Shimaya 1-chome, Konohana-ku**
**Osaka-shi, Osaka 554(JP)**
Inventor: **UMEMOTO, Misako c/o Osaka Works Sumitomo El. Ind.**
**Ltd. 1-3, Shimaya 1-chome Konohana-ku**
**Osaka-shi, Osaka 554(JP)**

(74) Representative: **Bankes, Stephen Charles Digby et al**
**BARON & WARREN 18 South End Kensington**
**London W8 5BU(GB)**

(54) **METHOD OF SEPARATING PROTEIN AND APPARATUS THEREFOR.**

(57) A method of precisely separating a specified protein from a solution of proteins in a short time, which comprises packing a gel, prepared by immobilizing a DNA having a sequence which specifically combines with a restriction enzyme *Eco* RI through bonding to a silica particle at its terminal, in a small-diameter chromatographic column (8), pouring a protein solution containing *Eco* RI into the column to combine the gel with *Eco* RI, pouring a mixture of two buffers into the column while giving thereto a concentration gradient with the lapse of time, eluting *Eco* RI when the concentration of the buffer reaches a specified value, developing the buffer eluted from the column on a nitrocellulose membrane, and taking out a portion containing *Eco* RI.

BACKGROUND OF THE INVENTION

1. Field of the invention

The present invention relates to a method and an apparatus for separating proteins, which is used in the field of molecular biology and the like for accurate and continuous separation of proteins which couple selectively with a sequence of nucleic acid.

2. Description of the Related Art

DNA-binding proteins have been separated in the prior art by using a separation method for substances comprising the steps of separating a protein solution by using an open column chromatography (affinity chromatography or gel filtration) which utilizes the difference in partition coefficients of proteins between the stationary phase and mobile phase, and collecting a portion of fractions of this solution with time in respective bottles like test tubes. As an example of this separation method for specific kinds of proteins (Prior Art 1), a protein Eco RI contained in a protein solution is made to couple selectively with a carrier (by incubating for 1 hour at 15°C ) by using a column chromatography (an affinity chromatography) comprising an immobilized synthetic DNA on an agarose series of Tresyl-activated Sepharose 4B (trade name of Pharmacia Co.) which is a soft carrier, followed by a selective elution of Eco RI with NaCl solution by flowing the solution with a change in NaCl concentration from 0.2M to 1.5M (Nucleic Acid Research, IRL Press Ltd., published in 1988, pages 10283 - 10299).

A soft-type of gel was used in the column in Prior Art 1 because soft gels such as cellulose and agarose are generally excellent in chemical stability and hydrophilic property and, moreover, are able to bear a large surface area. A method for fractionating substances in a sample solution by a gel electrophoresis has also been used (Prior art 2), wherein a designated band on the gel is cut-out after the electrophoresis. An alternative method for collecting a desired substance has also been used (Prior Art 3), wherein a portion of the sample solution is added into a centrifuge tube having a density gradient by sucrose and the tube is subjected to a centrifugal separation under a rotation of 10,000 to 80,000 rpm to fractionate the substances in the solution in the order of their density, followed by taking out the fractionated substances by using a syringe with a needle or the like.

Resolution is lowered in Prior Art 1 under a high hydrostatic pressure by a structural changes of the gel such as destruction of the gel structure, since so-called soft gels comprising soft carriers of polysaccharides such as cellulose or agarose are in general poor in physical strength. Under a low hydrostatic pressure, on the other hand, flow rate of the solution is lowered and an accurate density gradient is disturbed by the effect of diffusion of the substances once separated in the column, thereby making it difficult to maintain a high resolution and requiring a long separation time.

Use of more rigid silica or porous glass as carriers may be considered. These materials were, however, not applicable for practical uses because the amount of the immobilized DNA was generally too small when attempts were made to immobilize a DNA which coupled selectively with a specific kind of protein.

Use of a column with a small inner diameter may be considered for the purpose of minimizing the effect of diffusion. In this method, however, a high pressure was required to make the solution flow, which made the handling of the column difficult, and a solution having an accurate time-dependent density gradient was also difficult to realize.

When respective portions of a solution which are eluted out from the column and contain different kinds of proteins are collected in respective bottles in portions of predetermined volume (5 ml for example), and when a large amount of a specific kind of protein is contained in the solution, the protein is collected into separate bottles or is mixed with other kinds of proteins which are collected in the next bottle. Though mixing of the protein with different kinds of proteins could be avoided to some extent by making the volume of the fractionating bottles smaller, there was still a limitation in accurate separation and its operation was very troublesome.

An accurate separation of proteins was possible in Prior Art 2 but isolation of proteins from the band portions was very difficult. An operation in Prior Art 3 to take out the separated proteins by using a syringe needle requires to pay a careful attention, which was also difficult.

SUMMARY OF THE INVENTION

The present invention has been achieved to solve the problems in the above-described prior arts, providing a method and an apparatus for adsorbing and separating proteins accurately in a short period of time.

The essential object of the present invention is therefore to provide a method and an apparatus for separating proteins, which comprises a concentration-variable flow unit by which the solution is made to flow under a time dependent change in concentration, a separation unit of proteins to separate various kinds of proteins in the protein solution, a transport path of solutions for transporting the solution from the concentration-variable flow unit to the separation unit of proteins, and a receiving unit of proteins to receive the solution containing the proteins separated by the solution with time at the separation unit of proteins, and which is provided with a sample injection port to inject the sample solution of proteins at the separation unit of proteins or at the transport path of solutions, and a flow-out port of solutions to flow out the solution to the receiving unit of proteins, characterized in that the protein solution is supplied to the separation apparatus for proteins which is characterized in that the separation unit for proteins comprises an adsorbent in which nucleic acids which are able to couple selectively with a specific kind of protein are immobilized on a solid carrier having a sufficient amount of rigidity not to cause any destructive deformation by the pressure during the supply and passage of the solution, and the specific kind of protein contained in the sample solution of proteins is selectively adsorbed to the adsorbent, followed by dissociation of the specific kind of protein by supplying the solution to the adsorbent.

The invention is described hereinafter referring to the drawings.

BRIEF DESCRIPTION OF THE DRAWINGS

These and other objects and features of the present invention will become apparent from the following description taken in conjunction with the preferred embodiment thereof with reference to the accompanying drawings, in which:

Fig. 1 is a schematic illustration of the apparatus used in the present invention; and

Fig. 2 is an expanded partial diagrammatic view of the adsorbent in the present invention.

DETAILED DESCRIPTION OF THE INVENTION

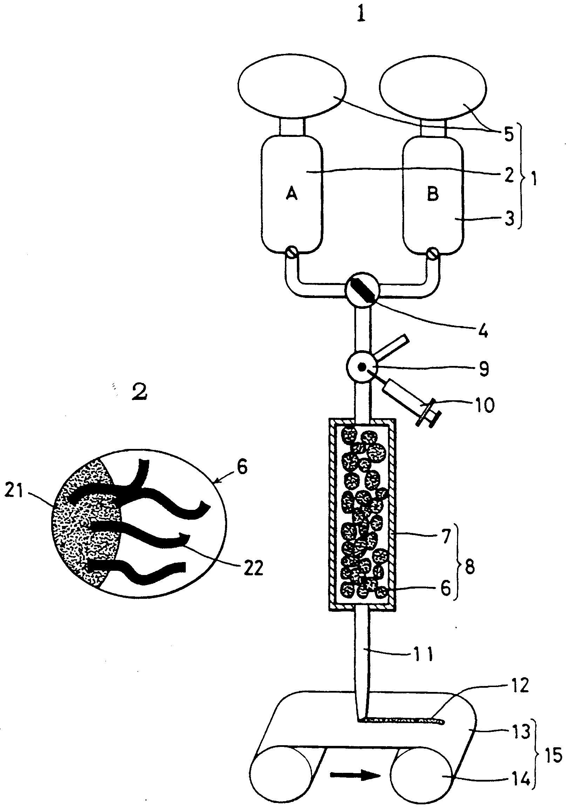

An apparatus constructed as illustrated in Fig. 1 can be used, for example, as a concentration-variable flowing unit which can flow the solvent with time-dependent changes in concentration, i.e. in composition, ionic strength or pH. In Fig. 1, the concentration-variable flowing unit 1 is composed of a bottle A 2 and bottle B 3 which contain a solvent and solute of the solution, respectively, which are able to dissociate the protein selectively adsorbed on the adsorbent 6 in the separation unit 8 for proteins, a mixing unit 4 for solutions by which the solution and solute from the bottle A 2 and bottle B 3, respectively, are mixed with each other by varying their mixing ratio, and a pump 5. Solvents with different concentration can be contained in the bottle A 2 and bottle B 3, respectively.

A sample solution of proteins can be injected from the sample injection port 9 into the column by using a syringe 10.

The separation unit 8 for proteins is composed, for example, of a column 8 in which a chromatographic tube 7 is filled with an adsorbent 6 to adsorb a specific protein. The adsorbents to be used for the column 8 of the separation unit for proteins are, for example, the materials of inorganic polymers (silica, porous glass and the like) or natural or synthetic organic polymers (eponite, melamine, polyetheretherketone and the like) having functional groups or being able to introduce functional groups to which nucleic acids can be immobilized by means of physical or chemical methods; or particles of solid carriers such as metals (titanium oxide and the like), ceramics (silicon nitride and the like) or crystalline plates (sodium chloride and the like) are subjected to a surface treatment to immobilize a nucleic acid, preferably at its molecular terminal, which is able to couple with a specific protein. Mechanical strength of the solid carrier should be large enough so that it can endure a hydrodynamic pressure of 10 $kg/cm^2$, preferably 100 $kg/cm^2$, during the supply and passage of the solution. A particle diameter of 10 $\mu$m or less is preferable, because a sufficient amount of surface area of the particles in the adsorbent is obtained when the value is less than 10 $\mu$m.

For example, in separating a restriction enzyme Eco RI contained in a sample solution of proteins by using the apparatus according to the present invention, the adsorbents to be used are those to which DNA's having the following sequences are bound and immobilized to the adsorbent:

(a) 5' X CGG ATA TAT <u>GAA TTC</u> GCG CG 3'

(b) 3' GCC TAT ATA <u>CTT AAG</u> CGC GC 5'

The binding site of the restriction enzyme Eco RI is the part underlined above.

The preparation method of the DNA affinity column by immobilizing the above-described DNA's to silica particles (5 $\mu$m in diameter) is, for example, as follows: After drying 1 $\mu$M of each purified DNA's having above-described sequences in vacuum, they are dissolved in 100 $\mu$l of 1M NaCl solution, respectively, and 100 $\mu$l of 0.4M NaHCO$_3$ (pH 7.5) is added to the solution. After a heat treatment for 2 minutes at 95 °C on a water bath, the solution is subjected to annealing within 20 minutes until the temperature is cooled to 55 °C.

100 milligrams of Tresyl activated silica gel is added to the solution and the mixture is allowed to react for 24 hours at room temperature. After the reaction has been completed, the gel is washed with 1M NaCl three times and a DNA immobilized adsorbent is obtained as a residue after centrifugation. The amount of the immobilized DNA can be readily determined by using UV light when light-transmittable materials like transparent solid resins (polyesters, acrylic resins and the like) or silica are used. This enables an accurate control of the amount of the immobilized DNA since the amount can be readily evaluated. The adsorbent is suspended in a buffer A (NaCl 0.5M, pH 7.5, EDTA 1mM, phosphoric acid 10mM) and placed in a pucker, and the chromatographic tube is filled with the suspension by flowing a buffer B (NaCl 150mM, phosphoric acid 10mM). The column is washed by flowing a buffer C (Tris-Hcl 40mM, pH 7.5, NaCl 0.2M, EDTA 5mM, DTT 2mM), thereby preparing a DNA affinity column.

The adsorbent 6 in the column is considered to be a configuration as illustrated in the expanded partial diagrammatic view in Fig. 2. Molecular terminals of DNA's 22 are bound and immobilized to the carriers 21 as supporting members.

The nucleic acid which is immobilized to the particles and couples selectively with a specific protein are not limited to DNA's synthesized and prepared by using a DNA synthesizer, but DNA's and RNA's from natural origins (plasmid or cDNA, for example) can also be used. The chromatographic tubes to be used for the columns are not limited particularly so far they endure the hydrostatic pressure of 100 kg/cm$^2$ and are made of an inert material to the solution. Metal tubes (stainless steel etc.), plastics (high-density polyethylene etc.) and inorganic materials (glass etc.) can also be used. Chromatographic tubes with their inner diameter of 4 mm$^2$ or less are preferable for the purpose of more accurate separation of proteins.

The reason why the separation is improved by using the above-described chromatographic tube according to the present invention is probably due to a smaller amount of diffusion of the separated proteins along the radial direction of the column when the column diameter is less than 4 mm$^2$. In particular, the inner diameter of 1 mm$^2$ or less is preferable since diffusion is suppressed by the viscosity of water.

The solution 12 is developed on the receiving unit 15 of proteins from the separation unit 8 (column) for proteins through the flowing-out port 11 of the solution.

The receiving unit 15 of proteins can be, for example, an apparatus by which the solution containing the separated proteins is developed successively on a membrane 13 by a rotor 14. The direction of the solution 12 comprising the solution developed on a flat plate can be a linear shape or a spiral shape, and it is not limited to a particular direction. Natural or synthetic organic polymer materials such as nitrocellulose, nylon, polytetrafluoroethylene or polyethylene, or inorganic polymers such as silicone resins and metals such as aluminum can be used for the materials of the membrane 13 in an apparatus in which the solution is developed on the membrane 13.

In the present invention, a specific kind of protein may be dissociated from the adsorbent when the temperature has reached to a predetermined temperature by a time-dependent change of the temperature of the adsorbent to which the specific kind of protein is selectively adsorbed.

(Example 1)

The buffer solution C contained in the bottle A 2 and a buffer solution D contained in the bottle B 3 (Tris-HCl 40mM, NaCl 1.5M, pH 7.5, EDTA 5mM, DTT 2mM) were mixed together so that a concentration gradient (a time dependent change in the concentration) of the buffer D is formed from 0% to 100% within 10 minutes by using a separation apparatus for proteins provided with a concentration-variable flow unit 1 comprising a bottle A 2, bottle B 3 and a mixing unit 4 of the solution, and the solution was flowed at a flow rate of 0.3 ml/min. and transferred to the separation unit 8 for proteins via a solvent transport unit 16.

The separation unit 8 for proteins used in the present invention is composed of a chromatographic tube 7 (made of stainless steel) of 2.1 mm in inner diameter and 100 mm in length, in which gels formed by immobilizing DNA's, which couple selectively with the restriction enzyme Eco RI, to silica gel having a particle size of 5 $\mu$m in diameter at their molecular terminals are packed.

The amount of the immobilized DNA was determined by diffusing the adsorbent into a saturated sucrose solution and measuring UV absorbance of DNA at 260 nm. The immobilized amount of the aforementioned synthetic DNA (a) (weight of synthetic DNA of 20 bases per one strand/weight of dried silica gel) was 0 to 3 mg/g.d.g.

Prior to transferring the above-described solution, a crude protein solution in the buffer solution C containing Eco RI is used as a sample solution of proteins and, after injecting 200 $\mu$l of the solution by a syringe from the sample injection port equipped at one end of the separation unit of proteins, Eco RI is made to bind selectively to the gel by allowing to react for 1 hour at 15°C. Eco RI was eluted out by flowing the above-described solution for 10 minutes through the separation unit 8 for proteins. The solution containing Eco RI was then made to flow out from the flowing-out port 11 of solution equipped at the other end of the separation unit 8 of proteins, followed by a development of the solution in the receiving unit 15 of proteins.

The receiving unit 15 of proteins provided in the present invention comprises a nitrocellulose membrane (manufactured by Ammersham Co.) as a membrane 13 which moves at a speed of 1.5 cm/min. The nitrocellulose membrane has a dimension of 50 mm in width and 300 mm in length which was wetted with a binding solution (HEPES: 10mM, NaCl: 50mM, MgCl$_2$: 10mM, EDTA: 0.1mM, DTT: mM and defatted powdered milk: 2.5%) prior to use.

Confirmation if Eco RI was separated or not was carried out by the following method.

A double-stranded DNA having a sequence to couple with Eco RI was synthesized and was labelled with $^{32}$P. The nitrocellurose membrane obtained in this example was immersed into a binding solution of 0.2M KCl containing $1 \times 10^5$ cpm/ml of this labelled DNA, and the DNA was allowed to react with proteins. After washing the membrane with a 0.2M KCl binding solution for 1 hour, DNA was detected by an autoradiography, thereby finding one radio-active spot.

The radio-active spot was subjected to extraction and Eco RI was separated. For identification of Eco RI, the separated protein was subjected to an assay by 10% SDS - polyacrylamide electrophoresis and the electrophoresis pattern was compared with that of Eco RI prepared by a separate experiment, thereby obtaining an identical pattern.

(Example 2)

Immediately after injecting 100 $\mu$l of sample of Eco RI (manufactured by Nippon Gene Co.) into a column similar with that used in Example 1, the column was eluted with buffer C and D under a gradient (D: 0→ 100%/30 min.) at a flow rate of 0.1 ml/min. A plunger pump (CCPD, manufactured by TOSO Co.) was used and the experiment was carried out at room temperature.

The eluent was monitored by UV absorption at 280 nm by using a spectrophotometer (UV-8000, manufactured by TOSO Co.), and was collected by 1 minutes of time mode fractionation. A sample of 3 $\mu$l of the solution in each fraction was allowed to react with 1 $\mu$g of λ -DNA (manufactured by Takara Co.) for 2 hours at 37°C in a solution of 50mM NaCl, 100mM of Tris-HCl (pH 7.5) and 7mM of MgCl$_2$ (total volume 50 $\mu$l). The existence of Eco RI was tested for each fraction from the digestive activity against λ -DNA by using 0.75% agarose gel electrophoresis, and an especially high concentration of Eco RI was confirmed to exist in the fraction of 0.8M of NaCl concentration.

Proteins other than Eco RI which can be detected by UV absorbance do not interact with the column and pass through the column without any adsorption since a carrier having less selective adsorption property was used, thereby indicating a selective separation of Eco RI from other proteins.

(Example 3)

DNA immobilized Tresyl-silica gel prepared by the same method as used in Example 1 was packed in a column made of stainless steel with a diameter of 1 mm and a length of 10cm.

The column was attached to $\mu$LC (made by Isaco Co.) and washed by sending the buffer C.

Twenty microlitres of Eco RI sample was injected to the column and the column was eluted with the buffer C and D under a gradient (D: 0→ 100%/5min.) at a flow rate of 0.05 ml/min for 5 minutes at room temperature.

The eluent was collected in portions of 25 $\mu$l by time mode fractionation. Each fraction was tested for the separation of Eco RI and a selective separation of Eco RI was confirmed.

The present invention makes it possible to separate a protein accurately in a very short time by using an adsorbent comprising a nucleic acid which couples with a specific kind of protein and is bound to a solid carrier at its molecular terminal, and by eluting out and developing the specific kind of protein bound to the adsorbent by a solution whose concentration changes with time. The amount of the solution to be used is smaller compared with that of prior arts.

Although the present invention has been fully described by way of example with reference to the accompanying drawings, it is to be noted here that various changes and modifications will be apparent to those skilled in the art. Therefore, unless otherwise such changes and modifications depart from the scope of the present invention as defined by the appended claims, they should be construed as included therein.

## Claims

1. A separation method for proteins, characterized in that a sample solution of proteins is supplied to an adsorbent in which a nucleic acid which couples selectively with a specific kind of protein and can dissociate said protein in a solution of specific concentration is immobilized on a solid carrier having a sufficient amount of rigidity not to cause any destructive deformation by pressure during supply and passage of solutions, and said specific kind of protein contained in said sample solution of proteins is selectively adsorbed to said adsorbent, followed by dissociation of said specific kind of protein by supplying a solution to said adsorbent.

2. A separation apparatus for proteins which comprises a concentration-variable flow unit by which the solution is made to flow under a time dependent change in concentration, a separation unit for proteins to separate various kinds of proteins in a solution of proteins, a transport path of solutions for transporting the solution from said concentration-variable flow unit to said separation unit for proteins, and a receiving unit for proteins to receive with time said solution containing the proteins separated by said solution at said separation unit for proteins, and which is provided with a sample injection port to inject the sample solution of proteins at said separation unit for proteins or said transport path of solutions, and a flow-out port of solutions to flow out the solution to said receiving unit for proteins, characterized in that it comprises an adsorbent to which nucleic acids which can selectively adsorb a specific protein is immobilized to a solid support having a sufficient amount of rigidity not to cause a destructive deformation of said separation unit for proteins by the pressure of supply and passage of said solution.

3. A separation apparatus for proteins according to Claim 2, wherein the separation unit for proteins is a column which utilizes a chromatographic tube having an inner cross sectional area of 4 mm$^2$ or less.

4. A separation apparatus for proteins according to Claim 3, wherein the inner cross sectional area of the chromatographic tube is 1 mm$^2$ or less.

5. A separation apparatus for proteins according to Claim 2, wherein a nucleic acid which can selectively adsorb a specific protein is immobilized to a solid support at its molecular terminal.

6. A separation apparatus for proteins according to Claim 2 or 5, wherein the solid support has a rigidity endurable to a hydrostatic pressure of 10 kg/cm$^2$.

7. A separation apparatus for proteins according to Claim 6, wherein the solid support has a rigidity endurable to a hydrostatic pressure of 100 kg/cm$^2$.

# INTERNATIONAL SEARCH REPORT

International Application No  PCT/JP91/01697

**I. CLASSIFICATION OF SUBJECT MATTER** (if several classification symbols apply, indicate all) [6]

According to International Patent Classification (IPC) or to both National Classification and IPC

Int. Cl[5]  C07K3/20, G01N30/48

**II. FIELDS SEARCHED**

| Minimum Documentation Searched [7] | |
|---|---|
| Classification System | Classification Symbols |
| IPC | C07K3/20, G01N30/48 |

Documentation Searched other than Minimum Documentation
to the Extent that such Documents are Included in the Fields Searched [8]

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| Y | JP, A, 1-72054 (Chromatokem Inc.), March 16, 1989 (16. 03. 89) & EP, A1, 295073 | 1-7 |
| Y | JP, A, 63-72699 (Diagen Institicot fur Molequrarbiorojitsucea-deagnostick GmbH.), April 2, 1988 (02. 04. 88) & EP, A1, 263934 & DE, A1, 3627063 | 1-7 |
| Y | JP, A, 58-223062 (Tosoh Corp.), December 24, 1983 (24. 12. 83), Figs. 2-1, 2-2 (Family: none) | 2-7 |
| A | Edited by Japan Biochemical Society "New Biochemical Experimental Lecture 1, Protein I - separation·purification·quality -" February 26, 1990 (26. 02. 90), Tokyo Kogaku Dojin (Tokyo) p. 241-244, 246-257 | 1-7 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| February 18, 1992 (18. 02. 92) | March 10, 1992 (10. 03. 92) |
| International Searching Authority | Signature of Authorized Officer |
| Japanese Patent Office | |